# EUROPÄISCHE PATENTSCHRIFT

(11) **EP 0 458 329 B1**
(45) Veröffentlichungstag und Bekanntmachung des Hinweises auf die Patenterteilung: **31.01.1996**
(21) Anmeldenummer: 91108346.7
(22) Anmeldetag: 23.05.1991
(51) Int. Cl.: C12Q 1/56, C12Q 1/37

(54) **Verfahren zum Nachweis von Plasminogen-Aktivatoren, deren inhibitoren oder Stimulatoren**
Method for the detection of plasminogen activators, their inhibitors or stimulators
Procédé pour la détection des activateurs de plasminogène leurs inhibiteurs ou leurs agents stimulateurs

(30) Priorität: 25.05.1990 DE 4016978
(43) Veröffentlichungstag der Anmeldung: 27.11.1991
(73) Patentinhaber: BEHRINGWERKE Aktiengesellschaft, D-35001 Marburg (DE)
(72) Erfinder: Keuper, Hermann, Dr., W-3552 Wetter (DE)

(56) Entgegenhaltungen:
- EP-A- 0 369 333
- EP-A- 0 374 908
- DE-A- 3 722 082
- BIOCHEMISTRY, Band 28, Nr. 10, 16. Mai 1989; R. MACHOVICH et al., Seiten 4517-
- 4522/
- JOURNAL OF BIOLOGICAL CHEMISTRY, Band 263, Nr. 32. 15. November 1988; G.-Y. SHI et al., Seiten 17071-17075/
- CLINICAL CHEMISTRY, Band 32, Nr. 3, März 1986; J. CHMIELEWSKA et al., Seiten 482-485/

## Beschreibung

Die Erfindung betrifft ein Verfahren zum Nachweis der funktionellen Aktivität von Plasminogen-Aktivatoren sowie deren Inhibitoren und Stimulatoren in biologischen Proben sowie Reagenzien für dieses Verfahren.

Die funktionelle Bestimmung von Plasminogen-Aktivatoren (PA), deren Inhibitoren (PAI) oder Stimulatoren im Plasma erhält zunehmend Bedeutung für die klinische Routinediagnostik. Dazu müssen schnelle und empfindliche Tests entwickelt werden, die die genannten Proteine in Konzentrationen von wenigen µg/l nachweisen können. Im Falle der PAI oder PA-Stimulatoren (z. B. Fibrin-Monomere und Fibrin(ogen)-Spaltprodukte für tPA) wird der Plasmaprobe üblicherweise eine definierte Menge an PA zugesetzt und deren Restaktivität oder stimulierte Aktivität gemessen.

Bekannt ist ein Verfahren (Drapier, J.D., Tenu, J.P., Lemaire, G., Petit, J.F., Biochimie 61(1979) 463-471), bei dem die Empfindlichkeit dadurch erhöht wurde, daß die Aktivität des PA über die Aktivierung von dem Testgemisch zugesetztem Plasminogen zu Plasmin bestimmt wird. Das entstandene Plasmin kann dann z. B. mit einem chromogenen Substrat nachgewiesen werden. In diesem Verfahren wird Glu¹-, Lys⁷⁸-Plasminogen oder ein Gemisch dieser Plasminogenvarianten eingesetzt.

Dieses Verfahren hat jedoch einen schwerwiegenden Nachteil darin, daß in Plasmaproben α₂-Antiplasmin enthalten ist, welches das entstehende Glu¹- oder Lys⁷⁸-Plasmin schnell und irreversibel inhibiert. Der störende Einfluß des α₂-Antiplasmins wird bei dem Verfahren durch Verdünnung und/oder Fällung beseitigt, was zu einer umständlichen Handhabung und zu hohem Zeitaufwand bei der Aktivitätsbestimmung führt. Folglich ist dieses Verfahren für die Routinediagnostik nur wenig geeignet. Alternativ kann α₂-Antiplasmin durch Oxidation inaktiviert werden, wodurch aber das Pipettieren eines zusätzlichen Reagenzes erforderlich ist (DE 37 22 082). Zudem ist die Anwendung der oxidativen Inaktivierung von α₂-Antiplasmin beschränkt auf Bestimmungsmethoden, in denen alle wesentlichen Komponenten deutlich weniger oxidationsempfindlich sind als α₂-Antiplasmin.

Beschrieben wurde auch ein Verfahren, (EP-A 0 374 908), in dem ein Gemisch aus undefinierten, proteolytischen Plasminogen Abbauprodukten eingesetzt wurde. MOROZ (Blood 58 (1), 1981) beschreibt Leukocytenesterase Abbauprodukte von Plasminogen, die nach Aktivierung durch Urokinase ein verändertes Verhalten gegenüber den bekannten Plasminogeninhibitoren in Plasma aufweisen.

Die der Erfindung zugrunde liegende Aufgabe bestand nun darin, ein Verfahren zur funktionellen Bestimmung von Plasminogenaktivator Aktivität und deren Modulation durch Inhibitoren oder Stimulatoren zu finden, das einfach und zuverlässig und auch auf automatischen Analysatoren durchgeführt werden kann.

Erfindungsgemäß wird nun ein Verfahren zur funktionellen Bestimmung von Plasminogenaktivator Aktivität und deren Modifikation durch Inhibitoren oder Stimulatoren in Körperflüssigkeiten vorgeschlagen, wobei als PA-Substrat Mini-Plasminogen eingesetzt wird.

Das entstandene Plasmin bzw. die Plasminvariante wird bevorzugterweise mit chromogenen Substraten nachgewiesen. Chromogene Plasminsubstrate sind dem Fachmann an sich bekannt (siehe z. B. KOLDE, H.-J. et al., Thrombosis and Haemostasis 56 (1986), Seite 155-159).

Verfahren zur Bestimmung der funktionellen Plasminogenaktivator Aktivität sind dem Fachmann an sich bekannt (siehe z. B. DE 37 22 082).

Bevorzugt ist ein Verfahren, wobei eine Probe eines biologischen Materials, bevorzugterweise Plasma, mit Mini-Plasminogen in einer Menge von 0.05 - 50 µmol/l, besonders bevorzugt 0.1 - 5 µmol/l, sowie einem chromogenen Plasminsubstrat (0.01 - 10 µmol/l), bevorzugterweise HD-Nva-CHA-Lys-pNA oder HD-Val-Leu-Lys-pNA, inkubiert wird. Zur Bestimmung von Inhibitoren oder Stimulatoren von PA können zusätzlich PA wie z. B. tPA oder Urokinase hinzugesetzt werden. Zur Auswertung wird bevorzugterweise die im Absorptionsmedium des freigesetzten Chromophors gemessene Absorption oder die Absorptionsänderung pro Zeiteinheit mit einer Kalibrierungskurve verglichen.

Ganz besonders bevorzugt sind die in den Beispielen angegebenen Ausführungsformen.

Mini-Plasminogen kann nach dem Fachmann an sich bekannten Verfahren, chemisch, enzymatisch oder gentechnisch hergestellt werden (siehe z. B. R. Machovich, W.G. Owen, Biochemistry 28 (1989) 4517 - 4522).

Mini-Plasminogen kann auch nach dem Fachmann an sich bekannten Verfahren direkt im Testansatz erzeugt werden.

Durch die Verwendung der erfindungsgemäßen Plasminogen-Variante entfällt eine Abreicherung oder Inaktivierung des α₂-Antiplasmins und die Bestimmungsmethoden können wesentlich vereinfacht werden. Verfahren zum Nachweis und zur Quantifizierung von Plasmin sind dem Fachmann an sich bekannt. Bevorzugterweise erfolgt die Bestimmung mit Hilfe von chromogenen Substraten, wie z. B. HD-Nva-CHA-Lys-pNA oder HD-Val-Leu-Lys-pNA.

Die Bestimmung kann kinetisch oder als Endpunktverfahren durchgeführt werden.

Die Bestimmung kann bei 10-40 °C, bevorzugt bei 20-40 °C, ganz besonders bevorzugt bei 37 °C erfolgen.

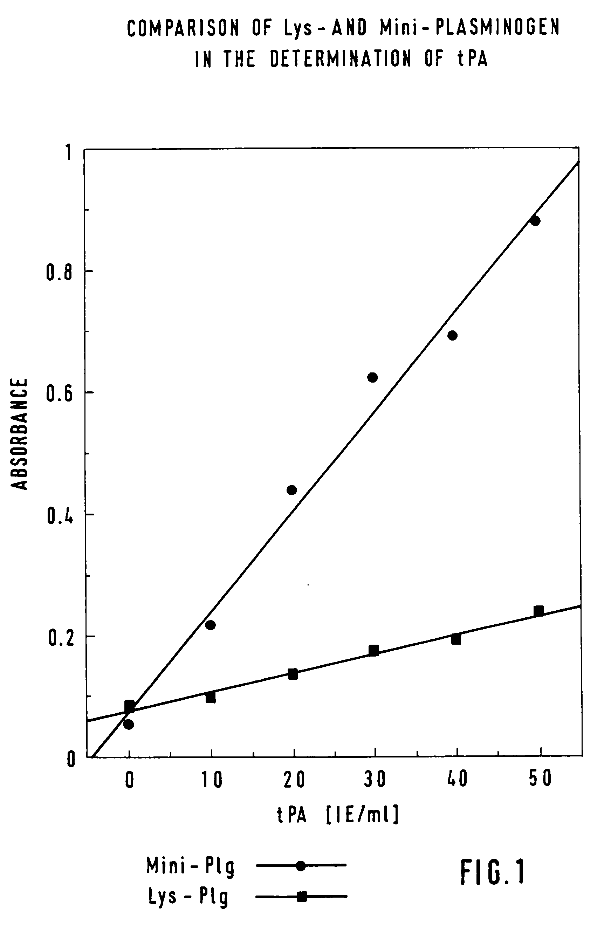

Gegenüber dem bekannten Stand der Technik (DE 37 22 082) zeichnet sich das erfindungsgemäße Verfahren durch seine Einfachheit und Sensitivität aus (siehe Fig. 1).

Die folgenden Beispiele dienen der Erläuterung des erfindungsgemäßen Verfahrens und sind in keiner Weise als Einschränkung zu betrachten.

### Beispiel 1

### Vergleich von Lys-Plasminogen und Mini-Plasminogen bei der Aktivitäts-Bestimmung von tPA

45µl eines Normal-Plasmapools wurden mit 50µl tPA in einer Konzentration von 0-50 IE/ml in HEPPS-Puffer (50mM N-(2-Hydroxyethyl)piperazin-N'-3-propansulfonsäure, 0,3% Triton X-100, pH 8,4) und 5µl Fibrin-Monomeren (0,8g/l in 1M KBr) gemischt. Anschließend wurden 100µl Lys-Plasminogen (2,3µM in HEPPS-Puffer) bzw. 100µl Mini-Plasminogen (1,1µM in HEPPS-Puffer) und 800µl Plasmin-Substrat (HD-Norvalyl-cyclohexylalanyl-lysyl-p-nitroanilid, 0,19 mmol/l in HEPPS-Puffer) zugegeben. Nach 30min Inkubation bei 37°C wurde die Reaktion mit 200µl 20% Essigsäure abgestoppt. Die Extinktion des freigesetzten p-Nitroanilins wurde bei 405nm gegen Wasser als Leerwert gemessen. Die gemessenen Extinktionen sind in Abb. 1 gegen die Aktivität des vorgelegten tPA aufgetragen. Für beide Plasminogenvarianten ergibt sich ein linearer Zusammenhang, doch ist die Steigung der Geraden und damit die Empfindlichkeit für Mini-Plasminogen mehr als 5fach höher als bei Verwendung von Lys-Plasminogen, welches zudem in mehr als doppelter Konzentration eingesetzt worden war.

### Beispiel 2

### Vergleich von Lys-Plasminogen und Mini-Plasminogen bei der Aktivitäts-Bestimmung von PAI

Plasma-Proben mit vorgegebenen PAI-Aktivitäten wurden durch Mischen der PAI-Standards S1 (0 E/ml) und S2 (5,9 E/ml) aus dem Testkit Berichrom^{R} PAI der Behringwerke AG hergestellt. 25µl der Plasma-Probe wurden 5min bei 37°C mit 50µl Urokinase in einer Konzentration von 5E/ml in TRIS-Puffer (100mM TRIS/HCl, 100mM NaCl, 1% Haemaccel^{(R)} (Behringwerke), 0,1% Triton X-100, pH 8,4) inkubiert. Anschließend wurden 100µl Miniplasminogen (6,3µM in TRIS-Puffer) zugegeben und wieder 5min bei 37°C inkubiert. Nach Zugabe von 250µl Plasmin-Substrat (0,6mM in 600mM NaCl/50mM TRIS/pH 8,4) und erneuter Inkubation (5min bei 37°C) wurde die Reaktion mit 50µl 50% Essigsäure abgestoppt. Die Extinktion des freigesetzten p-Nitroanilins wurde bei 405nm gemessen; für den Leerwert wurde anstelle der Urokinase TRIS-Puffer zur Probe zugesetzt. Zum Vergleich wurde mit Reagenzien der Behringwerke AG (Berichrom^{R} PAI) ein PAI-Aktivitätstest an den gleichen Proben durchgeführt. Der Test verläuft im wesenlichen wie oben beschrieben; anstelle des Mini-Plasminogens wird Lys-Plasminogen (7,3µM) verwendet, zur oxidativen Inaktivierung von störendem α₂-Antiplasmin in der Plasmaprobe muß Chloramin T zusammen mit dem Plasminogen zugegeben werden.

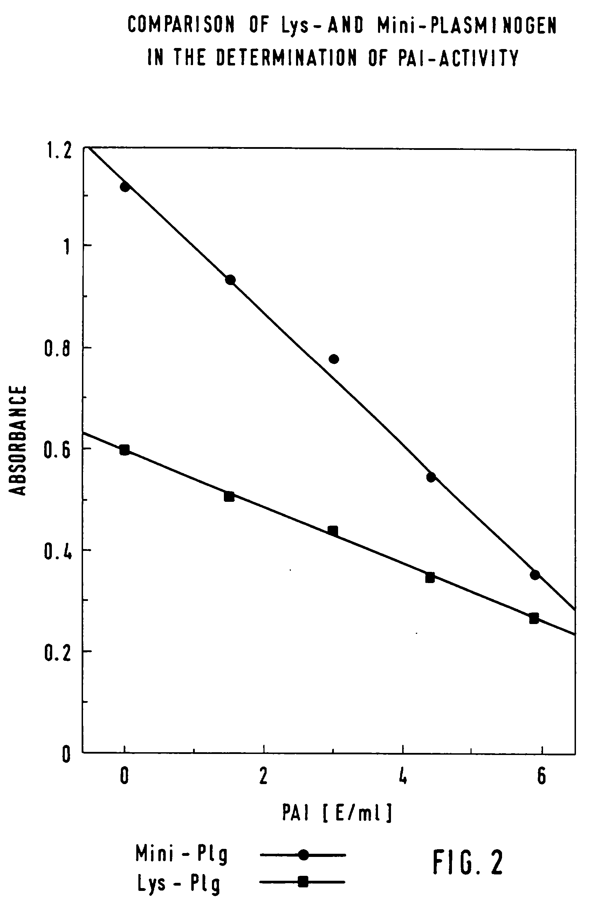

Die gemessenen Extinktionen sind in Fig. 2 gegen die vorgelegte PAI-Aktivität aufgetragen. Für beide Plasminogenvarianten ergibt sich ein linearer Zusammenhang. Im Gegensatz zum Experiment mit Lys-Plasminogen war aber bei der Messung der PAI-Aktivität mit Mini-Plasminogen das in der Probe enthaltene α₂-Antiplasmin nicht mit Chloramin T inaktiviert worden. Dennoch ist die Steigung der Geraden und damit die Empfindlichkeit im Falle des Mini-Plasminogens mehr als doppelt so hoch wie bei Verwendung von Lys-Plasminogen. Wird bei der Verwendung von Lys-Plasminogen kein Chloramin T zugegeben, so sind funktionelle PAI-Bestimmungen unter diesen Bedingungen nicht durchführbar.

## Patentansprüche

1. Verfahren zur funktionellen Bestimmung von Plasminogenaktivator (PA)-Aktivität sowie deren Modulation durch Inhibitoren und/oder Stimulatoren in Körperflüssigkeiten, wobei eine Probe des biologischen Materials mit einem Plasminogenaktivatorsubstrat inkubiert und die entstehende Plasminvariante bestimmt wird, dadurch gekennzeichnet, daß als Plasminogenaktivatorsubstrat Mini-Plasminogen eingesetzt wird.

2. Verfahren gemäß Anspruch 1, dadurch gekennzeichnet, daß die entstandene Plasminvariante mit einem chromogenen Substrat nachgewiesen wird.

3. Verfahren gemäß Anspruch 2, dadurch gekennzeichnet, daß eine Probe eines biologischen Materials, bevorzugterweise Plasma, mit Mini-Plasminogen (0.05 - 50 µmol/l), besonders bevorzugt 0.1 - 5 µmol/l sowie einem chromogenen Plasminsubstrat (0.01 - 10 µmol/l) bevorzugterweise HD-Nva-CHA-Lys-pNa oder HD-Val-Leu-pNa, inkubiert wird und zur Auswertung bevorzugterweise die gemessene Absorption oder Absorptionsänderung mit einer Kalibrierungskurve verglichen wird.

4. Verfahren gemäß Anspruch 1, dadurch gekennzeichnet, daß zur Bestimmung von Inhibitoren und/oder Stimulatoren der PA-Aktivität zusätzlich PA, wie z. B. t-PA oder Urokinase hinzugesetzt werden.

5. Verwendung von Mini-Plasminogen in einem Verfahren gemäß Anspruch 1.

## Claims

1. A method for the functional determination of plasminogen activator (PA) activity and the modulation thereof by inhibitors and/or stimulators in body fluids, where a sample of the biological material is incubated with a plasminogen activator substrate, and the resulting plasmin variant is determined, which comprises employing mini-plasminogen as plasminogen activator substrate.

2. The method as claimed in claim 1, wherein the resulting plasmin variant is detected with a chromogenic substrate.

3. The method as claimed in claim 2, wherein a sample of a biological material, preferably plasma, is incubated with mini-plasminogen (0.05 -50 µmol/l), particularly preferably 0.1 - 5 µmol/l, and with a chromogenic plasmin -substrate (0.01 -10 µmol/l), preferably HD-Nva-CHA-Lys-pNA or HD-Val-Leu-pNA, and evaluation is preferably carried out by comparing the measured absorption or change in absorption with a calibration plot.

4. The method as claimed in claim 1, wherein PAs such as, for example, t-PA or urokinase are additionally added for the determination of inhibitors and/or stimulators of PA activity.

5. The use of mini-plasminogen in a method as claimed in claim 1.

## Revendications

1. Procédé pour la détermination fonctionnelle de l'activité d'un activateur du plasminogène ainsi que des variations de ladite activité en présence d'inhibiteurs et/ou de stimulateurs dans les liquides corporels, dans lequel on incube un échantillon du matériau biologique avec un substrat activateur du plasminogène et on détermine les variations correspondantes de plasmide, caractérisé en ce que l'on utilise en tant que substrat activateur du plasminogène, du mini-plasminogène.

2. Procédé selon la revendication 1, caractérisé en ce que les variations résultantes de la plasmine sont mises en évidence à l'aide d'un substrat chromogène.

3. Procédé selon la revendication 2, caractérisé en ce qu'un échantillon du matériau biologique, de préférence un plasma, est incubé en présence de mini-plasminogène (0,05-50 µmol/l), plus préférablement 0,1-5 µmol/l ainsi que d'un substrat chromogène de la plasmine (0,01-10 µmol/l), de préférence HD-Nva-CHA-Lys-pNa ou HD-Val-Leu-pNa, et que l'on évalue graphiquement le résultat en comparant l'absorption ou la modification de l'absorption mesurée, avec une courbe de calibrage.

4. Procédé selon la revendication 1, caractérisé en ce que, afin de déterminer l'activité d'inhibiteurs et/ou de stimulateurs d'un activateur du plasminogène, on ajoute un activateur du plasminogène supplémentaire, tel que par exemple tPa ou l'urokinase.

5. Utilisation de mini-plasminogène dans un procédé selon la revendication 1.
